# EUROPEAN PATENT APPLICATION

(11) **EP 4 095 243 A1**
(43) Date of publication of application: **30.11.2022**
(21) Application number: 21175781.0
(22) Date of filing: 25.05.2021
(51) Int. Cl.: C12N 9/22, C12N 15/11

(54) **SYSTEM FOR HYBRIDIZATION-BASED PRECISION GENOME CLEAVAGE AND EDITING, AND USES THEREOF**

(71) Applicant: European Molecular Biology Laboratory, 69117 Heidelberg (DE)
(72) Inventor: Jann, Cosimo, 69117 Heidelberg (DE); Steinmetz, Lars Michael, 69117 Heidelberg (DE)
(74) Representative: Krauss, Jan

(57) **Abstract**

The present invention relates to the field of genome editing and in particular to a new proteinaceous system for hybridization-based precision genome cleavage and editing in a cell, tissue, and/or sample, as well as respective methods, systems and diagnostic and therapeutic uses thereof.

## Description

The present invention relates to the field of genome editing and in particular to a new proteinaceous system for hybridization-based precision genome cleavage and editing in a cell, tissue, and/or sample, as well as respective methods, systems and diagnostic and therapeutic uses thereof.

### Background of the invention

Genome editing is a type of genetic engineering in which DNA is inserted, deleted, modified or replaced in the genome of a living organism.

Targeted genome modification is a powerful tool that can be used to reverse the effect of pathogenic genetic variations and therefore has the potential to provide new therapies for human genetic diseases. Current genome engineering tools produce sequence-specific DNA breaks in a genome. The modification of the genomic sequence occurs at the next step and is the product of the activity of cellular DNA repair mechanisms triggered in response to the newly formed DNA break. Several approaches to genome editing have been developed, and currently available methods for programmable genome editing can be grouped into:
a) Protein-guided mega-nucleases, Zinc-Finger Nucleases (ZFNs) and Transcription factor Activator-Like Nucleases (TALENs) (reviewed in, for example, Adli, M. (2018). The CRISPR tool kit for genome editing and beyond. Nature Communications, 9 (1), 1-13);
b) RNA-guided CRISPR (Clustered Regularly Interspersed Short Palindromic Repeats) associated nuclease Class 1 systems, such as Cas9, Cas12a (Cpf1), Cas12b (C2c1) and Cas12e (CasX) (see, for example, Wu, J., Tang, B., and Tang, Y. (2020). Allele-specific genome targeting in the development of precision medicine. Theranostics, 10 (7), 3118), and the multicomponent Class 2 systems (see, for example, Makarova, K. S., Wolf, Y. I., Koonin, E. V. (2018). Classification and nomenclature of CRISPR-Cas systems: where from here? The CRISPR journal, 1(5), 325-336); and
c) DNA-guided argonaute (see, for example, Hegge, J. W., Swarts, D. C., Chandradoss, S. D., et al. (2019). DNA-guided DNA cleavage at moderate temperatures by Clostridium butyricum Argonaute. Nucleic acids research, 47(11), 5809-5821, or Anton Kuzmenko, Denis Yudin, Sergei Ryazansky, et al., Programmable DNA cleavage by Ago nucleases from mesophilic bacteria Clostridium butyricum and Limnothrix rosea, Nucleic Acids Research, Volume 47, Issue 11, 20 June 2019, Pages 5822-5836, https://doi.org/10.1093/nar/gkz379), and structure guided nucleases (see, for example, Xu, S., Cao, S., Zou, B., et al. (2016). An alternative novel tool for DNA editing without target sequence limitation: the structure-guided nuclease. Genome Biology, 17(1), 186).

For specific sequence recognition, ZFNs and TALENs rely on protein-DNA interaction, making their design laborious (e.g. involving protein engineering). Furthermore, they are not suitable for high throughput targeting of a large number of DNA target loci.

In CRISPR systems, sgRNAs with gene targeting sequences ranging between 18-24 nt in length (depending on the CRISPR nuclease) base-pair to a DNA target region that has been unwound from dsDNA into ssDNA through intrinsic helicase activity of CRISPR nucleases. The system thus requires the use and proper function of helicase or topoisomerase domains, and does not allow a design of RNAs with hybridization sequence parts of desired lengths (e.g. 15-20 nt in order to target multiple sequences at once comparable to the length of microRNAs and CRISPR gRNA targeting sequences, or up to more than 100 nt in order to bridge sequence repeats and enable specificity for highly repetitive target loci).

WO 2014/144288 discloses methods for increasing specificity of RNA-guided genome editing, e.g., editing using CRISPR/Cas9 systems, using RNA-guided FokI Nucleases (RFNs), e.g., FokI-dCas9 fusion proteins.

US 2010-055793A1 discloses a method of cleaving a gene of interest in a cell, the method comprising: providing a fusion protein comprising a zinc finger binding domain and a FokI cleavage domain, wherein the zinc finger binding domain binds to a target site in the gene of interest; and contacting the cell with the fusion protein under conditions such that the gene of interest is cleaved.

US 2020-0123542A1 discloses a composition comprising a non-naturally occurring RNA molecule which comprises a donor RNA covalently attached to a tracrRNA, which is in turn covalently attached to a guide RNA having homology to an intended DNA target site which contains a PAM recognition sequence. In the composition, a linker may connect the donor RNA to the tracrRNA, and the DNA target site is in eukaryotic genomic DNA. The composition can further comprise at least one mRNA encoding an RNA-guided DNA nuclease, at least one RNA-guided DNA nuclease, and the RNA-guided DNA nuclease can be a nickase and the RNA donor targets the same DNA strand that is targeted by the guide RNA.

Abudayyeh O.O., et al. ((2016) C2c2 is a single-component programmable RNA-guided RNA-targeting CRISPR effector. Science 353(6299):aaf5573) disclose that C2c2 is a programmable RNA-guided RNA-targeting CRISPR effector. Zhou et al (PPR-SMR protein SOT1 has RNA endonuclease activity, PNAS February 21, 2017 114 (8) E1554-E1563; first published February 6, 2017; https://doi.org/10.1073/pnas.1612460114) disclose that the protein SOT1 has RNA endonuclease activity. Compared with the CRISPR/Cas systems that use RNA-mediated base pairing to recognize RNA targets, SOT1 relies upon PPR-RNA interactions to recognize RNA targets. The results in this study reveal that SOT1 can achieve programmable RNA recognition and cleavage, suggesting that SOT1, similar to the CRISPR/Cas system, can be used as a tool for RNA manipulation.

It is an object of the present invention to provide a novel system for genome-editing, e.g. as an advantageous alternative to the above-mentioned CRISPR/Cas system. Other objects and advantages will become apparent upon further studying the present specification with reference to the accompanying examples.

In a first aspect thereof, the object of the present invention is solved by providing an RNA-guided nuclease system comprising i) at least one polypeptide comprising a sequence-unspecific endonuclease domain (sNUC), ii) at least one RNA-binding protein domain (RBPD), wherein the at least one sNUC polypeptide is complexed with and/or genetically fused to the at least one RBPD to form an sNUC-RBPD complex or fusion, and iii) at least one hybridizing RNA (hyRNA) comprising at least one RBPD-binding sequence that binds the RBPD of the sNUC-RBPD complex or fusion, and at least one hybridization sequence that binds to a desired target sequence. Preferably, the further system further comprises a donor DNA molecule comprising a donor DNA sequence flanked by DNA sequences that are homologous to the DNA sequences upstream and downstream to the target sequence. The present invention thus provides for a **Hy**bridization-based Precision Genome Cleavage and **Edit**ing (designated herein as "HyEdit"). In a preferred embodiment of the RNA-guided nuclease system according to the invention, the nuclease (sNUC) is replaced by an RNase in order to provide a hyRNA-RBPD-RNase system for targeted RNA-cleavage instead of DNA.

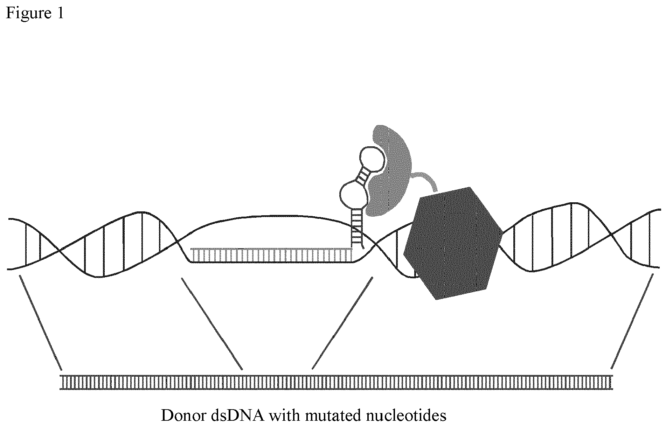

HyEdit is an artificial (recombinant) and customizable RNA-guided nuclease system. In one preferred embodiment, it can be used *in vitro* or *in vivo* as a programmable restriction enzyme, or *in vivo* or *in vitro* for high specificity precision genome editing (see, for example, Figure 1).

Furthermore, in another aspect of the invention an alternative inventive system is provided, wherein at least one sNUC domain is replaced with an effector protein, such as a fluorescent protein, or a transcriptional/translational repressor or activator. These effectors are fused to an RBPD in order to yield a small-size effector complex that can be recruited to an RNA or DNA sequence of interest via interaction with a hyRNA molecule, and therefore the system may be called recruitment system as well. In case of a fluorescent protein effector, this effector protein(s) is/are then used to produce a labelling read-out. In cases of transcriptional/translational repressor or activator proteins as effectors, transcription or translation is modulated, i.e. increased or decreased.

In this embodiment, the present invention provides an RNA-guided effector/recruitment system comprising:
i) at least one polypeptide selected from the group of a polypeptide comprising an effector polypeptide (EFF), such as, for example a fluorescent protein, and a transcriptional/translational repressor or activator polypeptide,
ii) at least one RNA-binding protein domain (RBPD), wherein the at least one EFF of i) is complexed with and/or genetically fused to the at least one RBPD to form an EFF-RBPD complex or fusion, and
iii) at least one hybridization RNA (hyRNA), comprising at least one RBPD-binding sequence that binds the RBPD of the EFF-RBPD complex or fusion, and a hybridization sequence that binds to a selected target sequence.

As further described herein, the HyEdit system is versatile and variable, and thus has many advantages compared with the CRISPR-system. A main use as disclosed herein in the embodiment hyRNA-RBPD-NUC complex including a donor-DNA sequence certainly is in genome-editing/gene therapy. Nevertheless, the system can be readily adapted for other uses, in other embodiments the nuclease (sNUC) can be replaced by an RNase (hyRNA-RBPD-RNase), for targeted RNA-cleavage, or the complex can comprise a detectable marker, such as a fluorescent protein in order to allow the detection of specific DNA-loci or RNA-molecules, as also explained further below.

In a preferred embodiment of the present invention, a programmable DNA cleavage by the system according to the present invention can be achieved with at least three components, namely
i) a sequence-independent or non-specific endonuclease domain (sNUC). Examples are the *Flavobacterium okeanokoites* FokI endonuclease domain, e.g. as used in Zinc-Finger Nucleases (ZFNs) (see, for example, Kim et al., 1996), and Transcription Activator-Like Nucleases (TALENs) (see, for example, Boch et al., 2009; Moscou et al., 2009), PvuII (see below), and other non-specific TypeIIS restriction enzyme nuclease domains, fused to or complexed with
ii) an RNA-binding protein domain (RBPD) specifically binding to an RNA sequence, such as, for example, the RNA-binding protein domain of the MS2 bacteriophage coat protein (MCP) binding to the MS2 RNA hairpin sequence (Peabody, 1993; Bertrand et al., 1998); or the PP7 bacteriophage coat protein (PCP) binding to the PP7 RNA hairpin sequence (Limet al., 2001; Lim and Peabody, 2002); or the GA bacteriophage coat protein (GCP) binding to the GA RNA hairpin sequence (Gott et al., 1991); or the Qβ bacteriophage coat protein (QCP) binding to the Qβ RNA hairpin sequence (Lim et al., 1996); or the bacteriophage lambda N peptide binding to the boxB hairpin sequence (Cai et al., 1998, van Gilst et al., 1997; Horiya et al., 2009); or Pumilio and FBF proteins binding to designated PUF binding sequences (Zamore et al., 1997; Cheong and Hall, 2006) or natural/artificial riboswitches (Saito et al., 2010) that are bound by a suitable protein ligand as their RBPD; and
iii) a hybridizing RNA molecule (hyRNA) bound to or binding to the RBPD. The hyRNA comprises an RBPD binding sequence, preferably a specific RBPD binding sequence (e.g. the MS2 RNA hairpin), and a hybridization sequence. The hybridization sequence may be of variable length and is sufficiently complimentary to a DNA target sequence (locus) in the genome of a target organism, a PCR fragment or in any other desired nucleic acid (DNA) molecule.

Suitable base-pairing of the hyRNA hybridization sequence to its DNA target sequence recruits the RBPD-sNUC complex to mediate dsDNA cleavage or nicking of a single strand of the dsDNA at the target locus. If no donor DNA is used (see below), the HyEdit complex will cleave the target DNA locus until repair results in alterations at the target locus which decrease hybridization to the hyRNA.

For a precise in vivo genome editing, the system comprises at least four components, i) to iii) as above, and
iv) a donor DNA sequence molecule (donor DNA) of a suitable altered or unaltered sequence, further including upstream and downstream flanking sequences that are sufficiently homologous to the sequences at the and/or surrounding the desired target locus, i.e. are located adjacent upstream and/or downstream thereof). The donor DNA serves as a template for homology-directed repair (HDR). For HDR in yeast, homology sequences of at least 20bp are recommended, and this number may differ in other organisms. The endogenous HDR machinery then conveys precise in vivo editing of the target DNA locus to result in an altered sequence as defined by the donor DNA. For efficient precision editing, the correctly edited sequence will afterwards no longer be targeted by the hyRNA-nuclease complex.

In one aspect of the inventive RNA-guided nuclease system according to the present invention, the at least one sNUC is an enzymatically inactive monomer able to form an enzymatically active dimer or multimer nuclease (d-sNUC), for example comprising two sNUCs when interacting with each other, such as when in close proximity to each other, when complexed with each other, and/or when genetically fused to each other. In this embodiment, the invention makes use of systems that combine two or more molecules, each with an inactive part of a dimer or multimer nuclease (d-sNUC) that becomes active, when functionally combined/interacting with each other based on the binding activities of the molecules.

In the RNA-guided nuclease system according to the present invention, the sNUC preferably comprises a non-specific TypeIIS restriction enzyme nuclease domain thereof, for example the FokI endonuclease domain. A preferred alternative for FokI is the PvuII restriction enzyme which has been successfully used in ZFNs (see, for example, Schierling B, Dannemann N, Gabsalilow L, Wende W, Cathomen T, Pingoud A. A novel zinc-finger nuclease platform with a sequence-specific cleavage module. Nucleic Acids Res. 2012;40(6):2623-2638. doi: 10.1093/nar/gkr1112).

In the RNA-guided nuclease system according to the present invention, the enzymatically active sNUC is capable of cleaving a double strand in the target sequence, or wherein only one sNUC of an sNUC dimer or multimer is enzymatically active, and can provide a nick in the target sequence, i.e. cleave only one of the strands of a double-stranded sequence. For example, manipulating the catalytic activity of individual monomers or the dimerization state of restriction endonucleases that recognize asymmetric sequences can result in nicking endonucleases.

Preferred is the RNA-guided nuclease system according to the present invention, wherein cleavage or nicking of the target sequence is independent from the recognition of a protospacer adjacent motif (PAM) in the target sequence. For a background on PAM, see, for example, Anders C, Niewoehner O, et al. (2014) Structural basis of PAM-dependent target DNA recognition by the Cas9 endonuclease. Nature, 513(7519):569-573; or Sternberg SH, Redding S, et al. (2014) DNA interrogation by the CRISPR RNA-guided endonuclease Cas9. Nature, 507(7490):62-67.

Also preferred is the RNA-guided nuclease system according to the present invention, wherein the sNUC-RBPD complex comprises an additional RBPD, wherein preferably the additional RBPD is complexed and/or genetically fused to the RBPD of the sNUC-RBPD complex. In these embodiments, the system therefore comprises two or more RBPDs. The RBPDs can be part of a dimer or multimer and can be identical (duplicated) or different. Furthermore, a helicase may be fused or conjugated to the sNUC-RBPD complex.

In the RNA-guided nuclease system according to the present invention, the RBPD can be selected from the group consisting of MS2 bacteriophage coat protein (MCP), PP7 bacteriophage coat protein (PCP), GA bacteriophage coat protein (GCP), Qβ bacteriophage coat protein (QCP), bacteriophage lambda N peptide, Pumilio protein, and an FBF protein or natural/artificial riboswitches.

In a preferred embodiment of the RNA-guided nuclease or recruitment system according to the present invention, the RBPD-binding sequence of the hybridization RNA is a hairpin sequence, and preferably the hairpin sequence is selected from the group consisting of an MS2 RNA hairpin sequence, a PP7 RNA hairpin sequence, a GA RNA hairpin sequence, a Qβ RNA hairpin sequence, a boxB hairpin sequence, and a PUF binding sequence.

In a preferred embodiment of the RNA-guided nuclease or recruitment system according to the present invention, the RBPD of the polypeptide/sNUC-RBPD binds to at least one hairpin forming sequence of the hyRNA as needed for targeted DNA cleavage, and preferably to between 2 and 10 hairpin forming sequences, more preferably between 3 and 8 hairpin forming sequences, and most preferred 5 or 6 hairpin forming sequences. More hairpins lead to the recruitment of additional RBPD-sNUCs. For DNA cleavage, this is advantageous for setups where a single hyRNA recruits dimer (or multimer) sNUC subunits. The preferred number of hairpins for DNA cleavage is 1-2. It is beneficial to increase the number of hairpins for other applications, e.g. to recruit many effector proteins (fluorescent proteins for labelling, transcriptional repressor or activator domains for regulating transcription).

In a preferred embodiment of the RNA-guided nuclease or recruitment system according to the present invention, the hybridization sequence of the hyRNA may be of variable length and is sufficiently complimentary to a DNA (or RNA) target sequence (locus) in the genome of a target organism, a PCR fragment or in any other desired nucleic acid (DNA, RNA) molecule, such as, for example, complimentary to 75% or more, 80% or more, 90% or more, 95% or more, such as 96, 97, 98, 99 or 100% (identical). In a preferred embodiment of the RNA-guided nuclease or recruitment system according to the present invention, the hybridization sequence of the hyRNA has a variable length of between at least 15 nt, preferably between 15 and 200 nt, more preferably between 15 to 25 nt, or between 100 to 200 nt. The desired lengths depend on the function, e.g. 15-20 nt in order to target multiple sequences at once, and thus comparable to the length of microRNAs and CRISPR gRNA targeting sequences, or up to more than 100 nt in order to bridge sequence repeats and enable specificity for highly repetitive target loci.

The stringency of hybridization is determined by the hybridization temperature and the salt concentration in the hybridization buffer, and high temperature and low salt is more stringent. A commonly used washing solution is SSC (Saline Sodium Citrate, a mixture of NaCitrate and NaCl). Hybridization may be carried out in solution or - more commonly - at least one component may be on a solid-phase support, e.g., nitrocellulose paper. Frequently used protocols employ a blocking reagent, such as casein from nonfat dried milk or bovine serum albumin, often in combination with denatured, fragmented salmon sperm DNA (or any other heterologous DNA of high complexity) and a detergent, such as SDS. Often a very high concentration of SDS is used as a blocking agent. Temperatures may be between 42 and 65 °C or higher, and buffers may be 3× SSC, 25 m*M* HEPES, pH 7.0, 0.25% SDS final. For in vitro applications, the temperature can also be raised and then slowly be lowered to allow specific annealing of hyRNA and target DNA. This is comparable to primer annealing in a PCR reaction. After reaching ambient temperature, the proteinaceous components can be added in order to avoid denaturation of the components due to the heat.

Another major advantage of the RNA-guided nuclease or recruitment system according to the present invention is the broad applicability thereof regarding the desired target sequences both *in vivo* and *in vitro.* Preferably the hybridization sequence is complementary to a target sequence selected from a genomic sequence, a PCR amplified nucleic acid fragment or another desired DNA or RNA molecule.

In another aspect of the present invention, the object of the invention is solved by a set of nucleic acid molecules, in particular isolated nucleic acid molecules, each molecule comprising or essentially comprising a sequence encoding for at least one polypeptide of the RNA-guided nuclease or recruitment system according to the present invention, and optionally furthermore encoding for at least one donor DNA molecule according to the present invention. In yet another aspect of the present invention, the object of the invention is solved by a nucleic acid vector or vector system comprising the nucleic acid molecules according to the present invention. The vector can comprise several molecules, and may be used in order to express, preferably recombinantly express, at least one polypeptide of the RNA-guided nuclease or recruitment system according to the present invention, and more preferably all polypeptides of a system as disclosed herein, i.e. sNUC-RBPD or EFF-RBPD, or the like, and, more preferably, at least one donor DNA. The vector can be a plasmid or other suitable vector, and is preferably an expression vector or other suitable expression nucleic acid construct in the chromosome of the cell, or on an extrachromosomal nucleic acid. More preferred is a set of nucleic acid molecules according to the present invention or the vector or vector system according to the present invention, further comprising at least one hyRNA molecule according to the present invention as described herein. The nucleic acids, such as the donor DNA can be produced in accordance with standard methods, e.g. synthetically produced, generated by in vitro replication, in vitro reverse transcription, and/or cloned into a plasmid. The nucleic acids, such as the donor DNA can be derived from naturally occurring sequences that are then modified in order to generate sequences in the molecule as desired. The nucleic acids, such as the donor DNA can further comprise additional modifications, such as labels or modified nucleotides, e.g. inosines, or the like.

In another aspect of the present invention, the object of the invention is solved by a cell, in particular a recombinant cell, comprising the RNA-guided nuclease or recruitment system according to the present invention, the set of nucleic acid molecules according to the present invention or the vector or vector system according to the present invention, and optionally at least one hyRNA molecule according to the present invention, wherein preferably the cell is a eukaryotic cell, preferably a mammalian cell or yeast cell. The cell preferably overexpresses a helicase, and/or a helicase is fused to the sNUC-RBPD polypeptide to raise efficiency.

In another aspect of the present invention, the object of the invention is solved by a method for nicking or cutting (cleaving) a selected target nucleic acid sequence in a cell, in vitro or in vivo, comprising the steps of: i) Providing a cell comprising the target sequence to be cleaved; ii) Introducing the RNA guided nuclease system according to the present invention into the cell; and binding the hybridization sequence of the hybridization RNA to the selected target sequence; and iii) Introducing of either a single-strand nick or a double-strand cut in the selected target sequence based on the activated at least one sNUC of the RNA guided nuclease system. In a preferred embodiment of the method according to the invention, the nuclease (sNUC) is replaced by an RNase in order to provide a hyRNA-RBPD-RNase system for targeted RNA-cleavage instead of DNA. Preferably, the cell is a eukaryotic cell, such as a mammalian cell or yeast cell.

In another aspect of the present invention, the object of the invention is solved by a method for in vivo or in vitro genome editing a selected target nucleic acid sequence in a cell, comprising the steps of: i) Providing a cell comprising the target sequence to be edited; ii) Introducing the RNA guided nuclease system according to the present invention into the cell; and binding the hybridization sequence of the hybridization RNA to the selected target sequence; iii) Introducing of either a single-strand break or a double-strand break into the selected target sequence based on the activated at least one sNUC of the RNA guided nuclease system; and either iv) Editing the single-strand break or double-strand break in the selected target sequence without the presence of a DNA donor template comprising a non-homologous end joining (NHEJ), or v) Editing the single-strand break or double-strand break in the selected target sequence with the presence of a DNA donor template comprising homologous recombination (HR). Preferably, the cell is a eukaryotic cell, preferably a mammalian cell.

In yet another aspect of the present invention, the object of the invention is solved by a method for in vitro or in vitro labelling of a selected target sequence comprising the steps of: i) Providing a cell comprising the target sequence to be labelled; ii) Introducing the RNA guided recruitment system according to the present invention into the cell; iii) Binding the hybridization sequence of the hybridization RNA to the selected target sequence; and iv) Detecting said binding to the selected target sequence based on the effector as recruited to said selected target sequence, wherein preferably the signal as detected is selected from a fluorescence signal and/or the activity of an enzyme, for example conversion of a specific substrate.

The detection of the binding and/or cleavage product based on the target nucleic acid can be direct (e.g. detecting a size change of the cleaved nucleic acid) or indirect using suitable fluorophores with or without quenchers. Cleaving the nucleic acid may release a fluorophore that can be detected, passing the cleaved nucleic acid through a nanopore sequencer detects changes, a fluorescently labeled nuclease could be localized, and/or the cleaving affects electrical currents that can be detected.

Fluorimetric readout can be achieved with a 384 well plate reader, NanoDrop, QuBit or by exposing tube under UV light (whole sample fluorescence), or by high-throughput microscopy with >1 µl volume in thin focal plane (single molecule detection is possible, since there are two signals per viral RNA molecule).

The detection of the cleaved product can also include a quantitative detection, i.e. the amount of fragment(s) as detected is used to determine the amount of selected target nucleic acid sequence in said cell, tissue and/or sample. In yet another aspect of the methods according to the present invention, multiple labels and/or markers are used. Markers can be used both for the nucleic acid molecules that form part of the assays, as well as the protein components (e.g. nuclease and/or fusions). Labels and markers can be included into the components of the assays (in particular the nucleic acids and/or the proteins), as well as constitute moieties that are attached, either covalently or non-covalently.

Detection of binding can be indirect, based on modulating the expression of a reporter gene. An at least one catalytically-dead nuclease can directly repress transcription by binding the promoter or coding region of the reporter gene. Alternatively, the nuclease can recruit regulatory domains responsible for the local modulation of gene expression, such as domains that upregulate expression (e.g. VP16, VPR) or downregulate expression (e.g. KRAB). The domains can be operably linked to the at least one catalytically-dead (inactive) nuclease. The domains can be recruited through interactions with binding domains fused to the at least one catalytically-dead nuclease.

In yet another aspect of the present invention, the object of the invention is solved by a method for in vitro or in vitro detecting of a selected target nucleic acid sequence of interest in a cell or sample, comprising the following steps of: i) Providing a cell or sample comprising the nucleic acid to be detected; ii) Adding or introducing a first RNA guided recruitment system according to the present invention, wherein said effector protein comprises a non-active bait protein sequence, and wherein the hybridization sequence of the hybridization RNA binds to the selected target sequence of the nucleic acid to be detected; iii) Adding or introducing a second RNA guided recruitment system according to the present invention, wherein said effector protein comprises a non-activate prey protein sequence, and wherein the second hybridization RNA binds to the selected target sequence of the nucleic acid to be detected and at least one sequence upstream and/or downstream of the selected target sequence, preferably directly or in close proximity to the selected target sequence; and iv) Detecting of the selected target sequence of interest in the cell or sample if a signal based on an interaction of the bait and the prey protein sequence is detected, wherein preferably the signal as detected is selected from a fluorescence signal and/or the activity of an enzyme, for example conversion of a specific substrate.

In a preferred embodiment of the methods according to the present invention, said at least one selected target nucleic acid sequence of interest is derived from a virus selected from Zika virus, human immunodeficiency virus (HIV), hepatitis B virus, hepatitis C virus, herpes virus, coronavirus, betacoronavirus, SARS, SAR CoV2, influenza, herpes simplex virus I, herpes simplex virus II, papillomavirus, rabies virus, cytomegalovirus, human serum parvo-like virus, respiratory syncytial virus, varicella-zoster virus, measles virus, adenovirus, human T-cell leukemia viruses, Epstein-Barr virus, murine leukemia virus, mumps virus, vesicular stomatitis virus, Sindbis virus, lymphocytic choriomeningitis virus, wart virus, blue tongue virus, Sendai virus, feline leukemia virus, reovirus, polio virus, simian virus 40, mouse mammary tumor virus, dengue virus, rubella virus, west Nile virus, and yellow fever virus.

In a preferred embodiment of the methods according to the present invention, said at least one selected target nucleic acid sequence of interest is derived from a pathogenic bacterium selected from *Mycobacterium tuberculosis, Streptococcus agalactiae,* methicillin-resistant *Staphylococcus aureus, Legionella pneumophila, Streptococcus pyogenes, Escherichia coli, Neisseria gonorrhoeae, Neisseria meningitidis, Pneumococcus, Cryptococcus neoformans, Treponema pallidum,* Lyme disease *spirochetes, Pseudomonas aeruginosa, Mycobacterium leprae,* and *Brucella abortus.*

In a preferred embodiment of the methods according to the present invention, the at least one selected target nucleic acid sequence of interest is derived from a gene the transcription and/or expression thereof is modified in response to external factors, such as, for example, metabolic factors or signals, hormones, pathogens, toxins, drugs, ageing and/or biotic or abiotic stress.

In a preferred embodiment of the method according to the present invention, the selected target sequence is present in the chromosome of the cell, or on an extrachromosomal nucleic acid, such as, for example, a plasmid or other vector, and wherein said sequence is preferably heterologous to said cell. Other examples are sequences, e.g. as introduced into a cell by, pathogens, like viruses or bacteria, or mutated sequences causing genetically based diseases, such as, for example, cancer.

In a preferred embodiment of the methods according to the present invention, as mentioned above as well, the RBPD-binding sequence of the hybridization RNA comprises a hairpin forming sequence as needed for targeted DNA cleavage, and preferably to between 2 and 10 hairpin forming sequences, more preferably between 3 and 8 hairpin forming sequences, and most preferred 5 or 6 hairpin forming sequences. More hairpins lead to the recruitment of additional RBPD-sNUCs. For DNA cleavage, this is advantageous for setups where a single hyRNA recruits dimer (or multimer) sNUC subunits. The preferred number of hairpins for DNA cleavage is 1-2. It is beneficial to increase the number of hairpins for other applications, e.g. to recruit many effector proteins (fluorescent proteins for labelling, transcriptional repressor or activator domains for regulating transcription).

Preferably, all methods according to the present invention can be automated, i.e. can be performed in high throughput, and/or on a chip or in wells of microtiter plates.

In yet another aspect of the present invention, the object of the invention is solved by a use of the RNA-guided nuclease system according to the present invention for nicking or cutting a selected target nucleic acid sequence in a cell. In yet another aspect of the present invention, the object of the invention is solved by a use of the RNA-guided nuclease system according to the present invention for in vivo or in vitro genome editing of a selected target nucleic acid sequence in a cell. In yet another aspect of the present invention, the object of the invention is solved by a use of the RNA-guided recruitment system according to the present invention in vitro or in vitro labelling of a selected target sequence or for in vitro or in vitro detecting of a selected target nucleic acid sequence of interest in a cell or sample.

In another aspect of the present invention, the object of the invention is solved by a kit, comprising the RNA-guided nuclease or recruitment system according to the present invention, for example in the form of polypeptides, a set of nucleic acid molecules, in particular isolated nucleic acid molecules, according to the present invention, and optionally furthermore at least one donor DNA molecule according to the present invention. The kit can furthermore include vectors that may be used in order to express, preferably recombinantly express, at least one polypeptide of the RNA-guided nuclease or recruitment system according to the present invention, and more preferably all polypeptides of a system as disclosed herein, i.e. sNUC-RBPD or EFF-RBPD, or the like. The at least one donor DNA as used with sNUC-RBPD can be reverse transcribed, or added by transfection using e.g. electroporation or viruses, such as AAV and lentivirus. More preferred is a kit according to the present invention, further comprising at least one hyRNA molecule according to the present invention. The kit may further include suitable buffers, media, and at last one recombinant cell comprising the set of nucleic acid molecules according to the present invention or the vector or vector system according to the present invention, and optionally at least one hyRNA molecule according to the present invention, wherein preferably the cell is a eukaryotic cell, preferably a mammalian cell.

Yet another aspect of the present invention then relates to a method for treating a disease or medical condition in a cell, tissue or organism, such as a mammal, preferably a human, wherein said condition is related to the presence of, expression of and/or mutation(s) in at least one selected target nucleic acid sequence of interest in a cell or sample. The method comprises providing a suitable treatment to said cell, tissue or organism, in particular a specific medical treatment, performing the method according to the invention as above, and modifying said treatment of said disease or medical condition based on the presence of, expression of and/or mutation(s) in said at least one selected target nucleic acid sequence of interest as detected. The medical conditions that can be detected using the present invention are those that are related to the at least one selected target nucleic acid sequence of interest. As explained above, the selected target nucleic acid sequence of interest can either itself constitute the origin of the condition or disease, for example in case of infections, e.g. viral, bacterial and/or fungal infections of the cells, tissues and/or samples as tested. Other conditions may relate more indirectly to the at least one selected target nucleic acid sequence of interest, for example in case of a nucleic acid that is aberrantly transcribed (present or found), expressed, processed and/or mutated. The selected target nucleic acid sequence of interest can be present in an increased or decreased amount when compared to a healthy control (e.g. a control based on a group of healthy or diseased samples). In the course of the treatment, the methods/system of the invention provide a, for example, quantitative readout of specific target nucleic acid sequence of interest in the patient sample that would directly inform about the course and effect/success of the treatment. The attending physician will then adjust the treatment accordingly, i.e. provide more with antiviral chemotherapeutics and/or biologics, if required. This treatment schedule can be repeated, if required. One conceivable treatment strategy would be to collect cells from a patient or group of patients, use precision genome editing to remove at least one disease causing malicious mutation, confirm the editing (including specificity, i.e. that no other non-intended changes were made to the DNA of the cells), grow and multiply the edited cells, and re-implant them into the patient.

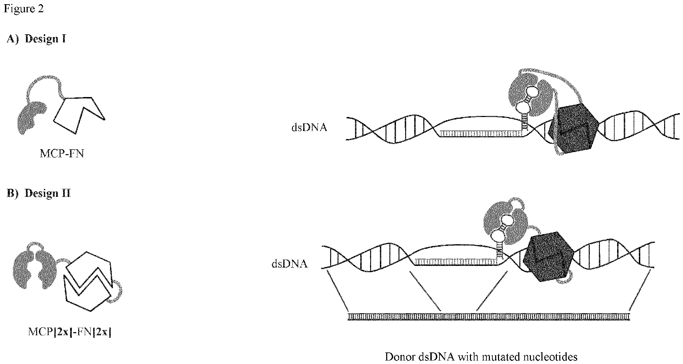

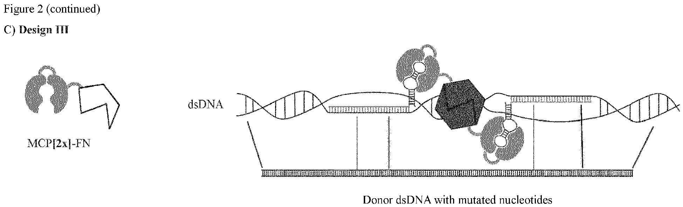

When compared to ZFNs and TALENs, the inventive HyEdit embodiments as described in Figure 2 make use of the sequence-non-specific FokI endonuclease domain which also has been successfully used in ZFNs and TALENs. Optimized FokI variants and homo-/heterodimer assemblies for dsDNA cleavage or nicking can be directly implemented in HyEdit systems. ZFNs and TALENs are based on protein-DNA interaction for specific sequence recognition, making their design laborious (involves protein engineering), and not suitable for high throughput targeting of a large number of DNA target loci.

Comparable to the highly specific two-component systems of ZFNs and TALENs, variations of HyEdit systems can consist of two components, and advantageously FokI dimerization and activation is then only occurring when these two components are in close physical proximity, i.e. two hyRNA-RBPD-FokI complexes binding two distinct target DNA sequences in very close physical proximity.

When compared to CRISPR nucleases, the inventive HyEdit embodiments conveniently rely on RNA base-pairing of the hyRNA with a complementary DNA target sequence, comparable to the single guide RNA (sgRNA) employed with CRISPR-based nucleases. The design and synthesis of oligonucleotides for hyRNAs or hyRNA-encoding DNA is therefore relatively simple and comparable to guide RNA design of CRISPR-based systems. In CRISPR systems, sgRNAs with gene targeting sequences ranging between 18-24 nt in length (depending on the CRISPR nuclease) base-pair to a DNA target region that has been unwound from dsDNA to ssDNA through intrinsic helicase activity of CRISPR nucleases. In contrast, HyEdit systems are artificially designed in a minimalistic fashion and do not require helicase or topoisomerase domains, and further allow design of hyRNA hybridization sequence lengths with arbitrary length (e.g. 15-20 nt to target multiple sequences at once comparable to the length of microRNAs and CRISPR gRNA targeting sequences, or up to more than 100 nt to bridge sequence repeats and enable specificity for highly repetitive target loci). The inventive HyEdit system can, similarly to CRISPR-based systems, be used for in vivo editing in high-throughput, e.g. for functional genetics screens, by using libraries of different hyRNAs. Importantly, HyEdit does not rely on any components related to CRISPR-based systems, and offers several advantages over CRISPR nucleases as follows.
I. Sequence independency: HyEdit is independent of sequence motifs, such as the Protospacer-Adjacent Motif (PAM) requirements of all CRISPR nucleases. HyEdit systems are artificially designed and independent of any CRISPR system components.
II. Extremely high specificity: High-specificity HyEdit systems rely on the specific binding of two independent (e.g. MS2-FokI) components at distinct DNA sequences of the same genomic locus. Close physical proximity enables dimerization of the two FokI domains which is required to activate nuclease activity. This binding requirement of two parts makes the cleavage and editing specificity of HyEdit highly specific to the defined region. Recently, the FokI nuclease domain has been fused to catalytically inactive dCas9 to generate a system with high cleavage specificity which requires two dCas9-FokI proteins to bind at a certain DNA locus for cleavage (similar to ZFNs, TALENs, and the two-component HyEdit system). However, all CRISPR nucleases rely on PAM sequences (NGG in the case of the used spCas9). Cleavage with any dCRISPR-nuclease-Fokl system is therefore restricted to genomic loci that have two NGG PAM motifs within a particular distance of any target sequence, bearing huge restrictions in their application.
III. Size: The small size of HyEdit components allows for adenovirus (AAV) packaging and facilitated localization into organelle compartments. For example, the (non-optimized) MS2-FokI fusion protein (334 amino acids length and ∼42 kDa mass) is substantially smaller compared to the frequently used *Streptococcus pneumoniae* spCas9 (1368 amino acids, ∼158 kDa) and even size-optimized versions of Cas9, Cas12a and other CRISPR nucleases (minimal CRISPR nucleases include e.g. CjCas9, DbpCasX and PlmCasx which are composed of 984, 986 and 978 amino acids, respectively) (Wu et al., 2020).
IV. Customizable hybridization sequence length: In contrast to CRISPR systems that rely on dsDNA unwinding and pairing of guide RNAs with 18-24 nucleotides of target hybridization sequence, the inventive HyEdit system does not necessarily include a helicase enzyme, does not have unspecific binding as observed e.g. for fluorescently labelled Cas9 protein transiently binding to dsDNA (PAM scanning), and uses hyRNA molecules with hybridization sequences of basically arbitrary length. While short lengths of 10-15 nt enable targeting of multiple loci at the same time, extending the hybridization sequence allows to increase binding specificity, e.g. only to the targeted DNA locus. Using hybridization sequences of up to a hundred nucleotides allows for a precise targeting of, for example, highly repetitive DNA sequences through hyRNA extension into non-repetitive regions.
V. Customizability of RBPD and sNUC components: HyEdit is highly customizable through alterations of the hyRNA, but also of the RBPD and sNUC components. Dimerization requirements for RBPD and sNUC components allow specialized applications, including high-specificity dsDNA cleavage or nicking, some of which are illustrated in Figure 2.
VI. Customizability for applications other than DNA editing and to target RNA: The hyRNA can base-pair with RNA. sNUC components can be substituted with: RNases (to enable RNA cleavage), RNA/DNA-unspecific nucleases (to enable combined RNA and DNA cleavage), translation-modulating factors (to modulate translation of a target RNA), transcription-modulating factors (to modulate transcription of a target gene), fluorescent proteins (to enable imaging of DNA loci or RNA molecules).

Further advantages and specific uses can be obtained when using sNUCs and/or RBPDs that dimerize or form multi-protein-complexes. For example, if the MS2 RNA-binding domain (MCP) is used as RBPD, two MCPs will have to form a dimer when binding to the MS2 RNA hairpin sequence (Golmohammadi et al, 1993). If an FokI endonuclease domain (FN) is used as sNUC, two FNs will have to dimerize in order to result in the desired active nuclease (Bitinaite et al, 1998).

Use of dimerizing components, such as MCP and FN, enables at least three HyEdit designs (see for example Figure 2) where covalent bonds and non-covalent bonds are indicated with "-" and "∼", respectively.

Preferred non-limiting embodiments and variations of the inventive HyEdit system (embodiments 5 to 12) are now briefly described, including the MS2 RNA-binding domain as exemplary RBPD (MCP), and the FokI endonuclease domain as exemplary sNUC (FN). The basic characteristic HyEdit complex comprises the hyRNA-RBPD-sNUC complex or fusion in order to mediate dsDNA cleavage or nicking, depending on the double- or single-strand nuclease activity of the employed sNUC. For application in eukaryotic cells which have a nucleus, at least one NLS (nuclear localization signal) can be fused to the Ms2-FokI protein.
Embodiment (5): (5a) A single FN is fused to a single MCP to generate an MCP-FN fusion protein. The MCP of two fusion proteins dimerize and bind a single MS2 hairpin sequence of a single hyRNA molecule, bringing together two FNs that dimerize, and result in the nuclease-active hyRNA∼(MCP-FN)[2x] complex. (5b) Here, two FNs are fused to each other and to two MCPs to generate an MCP[2x]-FN[2x] fusion protein. The two linked MCPs dimerize and bind an MS2 hairpin sequence of a single hyRNA molecule, the two FNs dimerize, resulting in the nuclease-active hyRNA∼MCP[2x]-FN[2x] complex. (5c) A single FN is fused to two MCPs to generate an MCP[2x]-FN fusion protein. This setup requires two hyRNAs, each of which bind one MCP[2x]-FN fusion protein. The hyRNAs have hybridization sequences complimentary to two DNA target sequences, preferably in less than 100 nucleotides distance (depending on linker lengths and applications). This results in two hyRNA∼MCP[2x]-FN complexes recruited in close physical proximity to each other at the same DNA target locus in order to enable FN dimerization and nuclease activation. This increases cleavage and editing specificity due to FN being inactive as a monomer and the requirement of two hyRNAs to specifically hybridize to different DNA sequences to enable dimerization of two FNs for nuclease activation.
Embodiment (6): Use of homo-dimers of catalytically active FokI domains enables dsDNA cleavage. Alternatively, a single DNA strand in the targeted dsDNA can be nicked (cleavage of a single DNA phosphor-diester bond within a dsDNA) when using FokI hetero-dimers that consist of one catalytically inactive and one catalytically active FN variant, respectively. Nicking with FokI hetero-dimers still requires dimerization of the two FNs for nuclease activity, although only a single of the two FNs in the dimer is active and mediates dsDNA nicking. Fokl-based dsDNA nicking systems are employed in ZFNs as mentioned in i) as above, and are reported with increased homology-directed repair (HDR) and decreased non-homologous end joining (NHEJ) activities for precise sequence mutation (Ramirez et al., 2012) and insertion (Wang et al., 2012).
Embodiment (7): Several variations of the FokI nuclease domain have been characterized in the context of ZFN and TALENs, e.g. to generate FNs with increased specificity for dsDNA cleavage or nicking (Miller et al., 2019). These FN variants can be directly used in HyEdit systems that use FNs.
Embodiment (8): Variants of many RBPDs, including MCP, have been characterized for imaging and other applications to generate e.g. MCP variants with varied affinities to the MS2 RNA hairpin structure (Lim et al., 1994) and disabled in aggregation propensity (LeCuyer et al., 1995). These MCP variants can be directly used in HyEdit systems that target MCP and MS2 hairpins.
Embodiment (9): The hyRNA can be designed with varying lengths of the hybridization sequence to allow specific base-pairing to repetitive sequences. In addition, incorporation of RNA-modifications into the hyRNA, such as locked nucleic acids (LNAs) (Obika et al., 1997), make it possible to increase base pairing affinities of hyRNA to its target DNA sequence (Singh et al., 1998). LNAs have been used in fluorescently labelled oligonucleotides to generate dsDNA invading probes for in vivo imaging applications (Sau et al., 2010 and 2013; Guenther et al., 2015). This property can directly be introduced into hyRNAs and used for editing applications. LNAs allow to achieve hyRNA hybridization with single nucleotide mismatch sensitivity due to their high affinity and melting temperature (Simeonov et al., 2002). This property is especially useful for *in vitro* applications, where temperature can be adjusted to control dsDNA strand melting and hybridization to hyRNAs. LNA modifications can be used that further protect hyRNAs from RNase activity.
Embodiment (10): Multiple pairs of RBPDs and their respective RNA-binding sequences, as listed in ii) as above, can be used in combination to achieve high cleavage and editing specificity, or mediate simultaneous cleavage at multiple DNA loci.
Embodiment (11): The system can be further modified to target RNA by using a hyRNA hybridization sequence that hybridizes with an RNA sequence of interest and an unspecific RNase replacing the sNUC. This allows a programmable RNA cleavage. Replacement of the sNUC with fluorescent proteins and/or suitable translation factors further enable imaging and translational regulation of targeted RNA molecules. Hybridizing MS2 DNA oligos can also be used in RNA pull-downs (co-immunoprecipitations). Fusions of MCP with GFP and other fluorescent proteins are commonly used to image RNA molecules with multiple, typically up to 24 directly encoded MS2 hairpins (see, for example, Bertrand, E., Chartrand, P., Schaefer, M., Shenoy, S. M., Singer, R. H., & Long, R. M. (1998). Localization of ASH1 mRNA particles in living yeast. Molecular cell, 2(4), 437-445; and Tutucci, E., Vera, M., Biswas, J., Garcia, J., Parker, R., & Singer, R. H. (2018). An improved MS2 system for accurate reporting of the mRNA life cycle. Nature methods, 15(1), 81-89). Indirect imaging of a hybridizing oligo that contains the MS2 hairpin (such as a hyRNA) is generally not used, since the fluorescent oligo would be detected independent of hybridization. However, splitGFP systems can be used that give strong signals only when two hyRNAs hybridize to the target RNA and bring the two splitGFP components in close proximity.
Embodiment (12): Applications beyond genome editing are possible when using the inventive system for a localization and recruitment of diverse effector proteins. Recruitment of transcriptional activators or repressors to target promoters can e.g. enable targeted modulation of gene expression, while recruitment of fluorescent proteins is useful for imaging specific DNA regions or molecules, or specific RNA molecules, e.g. in disease and viral infection diagnostics. Such applications have been successfully performed with CRISPR-based systems, using catalytically inactive CRISPR nucleases, fused to diverse effector proteins (Adli, 2018), and the present system can be readily adjusted to these functions.

Furthermore, an inventive system is preferred, wherein the sNUC domain is replaced with an effector protein, such as a fluorescent protein, or a transcriptional/translational repressor or activator. These effectors are fused to an RBPD in order to yield a small-size effector complex that can be recruited to an RNA or DNA sequence of interest via interaction with a hyRNA molecule.

In the context of the present invention, unless explicitly mentioned otherwise, the term "about" shall mean a value as given +/- 10%.

The present invention specifically relates to the following items:
Item 1. An RNA-guided nuclease or recruitment system comprising: i) at least one polypeptide selected from the group of a polypeptide comprising a sequence-unspecific endonuclease domain (sNUC), an effector polypeptide, such as, for example a fluorescent protein, and a transcriptional/translational repressor or activator polypeptide, ii) at least one RNA-binding protein domain (RBPD), wherein the at least one polypeptide, and preferably the sNUC, of i) is complexed with and/or genetically fused to the at least one RBPD to form a polypeptide-RBPD, preferably an sNUC-RBPD, complex or fusion, and iii) at least one hybridization RNA (hyRNA), comprising at least one RBPD-binding sequence that binds the RBPD of the polypeptide-RBPD, and preferably in the sNUC-RBPD complex or fusion, and a hybridization sequence that binds to a selected target sequence.
Item 2. The RNA-guided nuclease or recruitment system according to Item 1, further comprising a donor DNA molecule comprising a donor DNA sequence flanked by DNA sequences that are homologous to the DNA sequences upstream and downstream to the target sequence.
Item 3. The RNA-guided nuclease system according to Item 1 or 2, wherein the at least one sNUC is an enzymatically inactive monomer able to form an enzymatically active dimer or multimer nuclease (d-sNUC), for example comprising two sNUCs when interacting with each other, when complexed with each other, and/or when genetically fused to each other.
Item 4. The RNA-guided nuclease system according to any one of Items 1 to 3, wherein the sNUC comprises a non-specific TypeIIS restriction enzyme nuclease domain thereof, for example the FokI endonuclease domain.
Item 5. The RNA-guided nuclease system according to any one of Items 1 to 4, wherein the enzymatically active sNUC is capable of cleaving a double strand in the target sequence, or wherein only one sNUC of an sNUC dimer or multimer is enzymatically active, and provides a nick in the target sequence.
Item 6. The RNA-guided nuclease system according Item 5, wherein cleavage or nicking of the target sequence is independent from the recognition of a protospacer adjacent motif (PAM) in the target sequence.
Item 7. The RNA-guided nuclease system according to any one of Items 1 to 6, wherein the sNUC-RBPD complex comprises an additional RBPD, wherein preferably the additional RBPD is complexed and/or genetically fused to the RBPD of the sNUC-RBPD complex.
Item 8. The RNA-guided nuclease or recruitment system according to any one of Items 1 to 7, wherein the RBPD is selected from the group consisting of MS2 bacteriophage coat protein (MCP), PP7 bacteriophage coat protein (PCP), GA bacteriophage coat protein (GCP), Qβ bacteriophage coat protein (QCP), bacteriophage lambda N peptide, Pumilio protein, and an FBF protein or natural/artificial riboswitches.
Item 9. The RNA-guided nuclease or recruitment system according to any one of Items 1 to 8, wherein the RBPD-binding sequence of the hybridization RNA is a hairpin sequence, wherein preferably the hairpin sequence is selected from the group consisting of MS2 RNA hairpin sequence, PP7 RNA hairpin sequence, GA RNA hairpin sequence, Qβ RNA hairpin sequence, boxB hairpin sequence, and PUF binding sequence.
Item 10. The RNA-guided nuclease system according to any one of Items 1 to 9, wherein the RBPD of the polypeptide/sNUC-RBPD binds to at least one hairpin structure of the hyRNA.
Item 11. The RNA-guided nuclease or recruitment system according to any one of Items 1 to 10, wherein the hybridization sequence of the hyRNA has a length of between at least 15 nt, preferably between 15 and 200 nt, more preferably between 15 to 25 nt, or between 100 to 200 nt.
Item 12. The RNA-guided nuclease or recruitment system according to any one of claims 1 to 11, wherein the hybridization sequence is complementary to a target sequence selected from a genomic sequence, a PCR amplified nucleic acid fragment or other desired DNA molecule.
Item 13. A set of nucleic acid molecules, each encoding for at least one polypeptide of the RNA-guided nuclease or recruitment system according to any one of Items 1 to 12, and optionally furthermore encoding for at least one donor DNA molecule according to any one of Items 2 to 12.
Item 14. A nucleic acid vector or vector system comprising the nucleic acid molecules according to Item 13.
Item 15. The set of nucleic acid molecules according to Item 13 or the vector or vector system according to claim 14, further comprising at least one hyRNA molecule according to any one of Items 1 to 12.
Item 16. A recombinant cell comprising the set of nucleic acid molecules according to Item 13 or the vector or vector system according to Item 14, and optionally at least one hyRNA molecule according to any one of Items 1 to 12, wherein preferably the cell is a eukaryotic cell, preferably a mammalian cell.
Item 17. A method for nicking or cutting a selected target nucleic acid sequence in a cell, comprising the steps of: i) Providing a cell comprising the target sequence to be cleaved; ii) Introducing the RNA guided nuclease system according to Item 1 into the cell; and binding the hybridization sequence of the hybridization RNA to the selected target sequence; and iii) Introducing of either a single-strand nick or a double-strand cut in the selected target sequence based on the activated at least one sNUC of the RNA guided nuclease system.
Item 18. A method for in vivo or in vitro genome editing a selected target nucleic acid sequence in a cell, comprising the steps of: i) Providing a cell comprising the target sequence to be edited; ii) Introducing the RNA guided nuclease system according to Item 1 or 2 into the cell; and binding the hybridization sequence of the hybridization RNA to the selected target sequence; iii) Introducing of either a single-strand break or a double-strand break into the selected target sequence based on the activated at least one sNUC of the RNA guided nuclease system; and either iv) Editing the single-strand break or double-strand break in the selected target sequence without the presence of a DNA donor template comprising a non-homologous end joining (NHEJ), or v) Editing the single-strand break or double-strand break in the selected target sequence with the presence of a DNA donor template comprising homologous recombination (HR).
Item 19. A method for in vitro or in vitro labelling of a selected target sequence comprising the steps of: i) Providing a cell comprising the target sequence to be labelled; ii) Introducing the RNA-guided recruitment system according to Item 1 into the cell; iii) Binding the hybridization sequence of the hybridization RNA to the selected target sequence; and iii) Detecting said binding to the selected target sequence based on the effector as recruited to said selected target sequence.
Item 20. A method for in vitro or in vitro detecting of a selected target nucleic acid sequence of interest in a cell or sample, comprising the following steps of: i) Providing a cell or sample comprising the nucleic acid to be detected; ii) Adding or introducing a first RNA guided recruitment system according to Item 1, wherein said effector protein comprises a non-active bait protein sequence, and wherein the hybridization sequence of the hybridization RNA binds to the selected target sequence of the nucleic acid to be detected; iii) Adding or introducing a second RNA guided recruitment system according to Item 1, wherein said effector protein comprises a non-activate prey protein sequence, and wherein the second hybridization RNA binds to the selected target sequence of the nucleic acid to be detected and at least one sequence upstream and/or downstream of the selected target sequence, preferably directly or in close proximity to the selected target sequence; and iv) Detecting of the selected target sequence of interest in the cell or sample if a signal based on an interaction of the bait and the prey protein sequence is detected, wherein preferably the signal as detected is selected from a fluorescence signal and/or the activity of an enzyme.
Item 21. The method according to any one of Items 17 to 20, wherein the selected target sequence is present in the chromosome of the cell, or on an extrachromosomal nucleic acid, such as, for example, a plasmid or other vector, and wherein said sequence is preferably a recombinant sequence.
Item 22. The method according to any one of Items 19 to 21, wherein the RBPD-binding sequence of the hybridization RNA comprises between 1 and 2 hairpins for use in editing, between 1 and 5 hairpin forming sequences for use with regulatory effectors, and between 1 and 20 hairpin forming sequences for use with labelling effectors.
Item 23. Use of the RNA-guided nuclease system according to any one of Items 1 to 12 for nicking or cutting a selected target nucleic acid sequence in a cell or an in vitro sample.
Item 24. Use of the RNA-guided nuclease system according to any one of Items 2 to 12 for in vivo or in vitro genome editing of a selected target nucleic acid sequence in a cell.
Item 25. Use of the RNA-guided recruitment system according to any one of Items 1 to 12 in vitro or in vitro labelling of a selected target sequence or for in vitro or in vivo detecting of a selected target nucleic acid sequence of interest in a cell or sample.

The present invention will now be further described in the following examples with reference to the accompanying Figures, nevertheless, without wanting to be limited thereto. For the purposes of the present invention, all references as cited herein are incorporated by reference in their entireties.

Figure 1 shows a schematic overview of the RNA-guided nuclease system according to the invention with a polypeptide comprising a sequence-unspecific endonuclease domain (sNUC) (hexagonal shape), the polypeptide comprising the RNA-binding protein domain (RBPD), fused/linked to the sNUC, and the hybridization RNA (hyRNA) bound to the RBPD-domain (in form of a hairpin structure), and the hybridization sequence bound to the selected target nucleic acid sequence. Further shown is a donor DNA molecule comprising a donor DNA sequence comprising mutations (not marked) flanked by DNA sequences that are homologous to the DNA sequences upstream and downstream to the target sequence.

Figure 2 shows preferred exemplary HyEdit designs A-C (I-III) according to the present invention, with the FokI nuclease domain (FN) as sNUC, the MS2 coat protein (MCP) as RBPD, and the MS2 RNA hairpin as RBPD-binding sequence. A) In a first design, a single FN is fused to a single MCP to generate an MCP-FN fusion protein. The MCP of two fusion proteins dimerize and bind a single MS2 hairpin sequence of a single hyRNA molecule, bringing together two FNs that dimerize, and resulting in the nuclease-active hyRNA∼(MCP-FN)[2x] complex. B) Design II: In a second design, two FNs are fused to each other and to two MCPs to generate an MCP[2x]-FN[2x] fusion protein. The two linked MCPs dimerize and bind an MS2 hairpin sequence of a single hyRNA molecule, and the two FNs dimerize, resulting in the nuclease-active hyRNA∼MCP[2x]-FN[2x] complex. C) Design III: In a third design, a single FN is fused to two MCPs to generate an MCP[2x]-FN fusion protein. This setup requires two hyRNAs, each of which bind one MCP[2x]-FN fusion protein. The hyRNAs have hybridization sequences complimentary to two DNA target sequences in less than 100 nucleotides distance (depending on linker lengths and application). This results in two hyRNA∼MCP[2x]-FN complexes recruited in close physical proximity to each other at the same DNA target locus to enable FN dimerization and nuclease activation. This increases cleavage and editing specificity due to FN being inactive as a monomer and the requirement of two hyRNAs to specifically hybridize to different DNA sequences to enable dimerization of two FNs for nuclease activation.

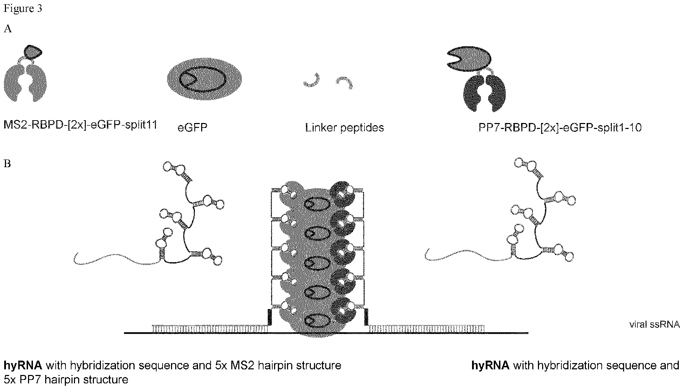

Figure 3 shows a preferred embodiment of the hybridization-based detection of nucleic acids according to the invention. A) Split eGFP components; B) Specific hybridization of two hyRNAs results in a strong single signal per molecule, based on split enhanced GFP (schematic depiction). The system requires two probes to specifically hybridize.

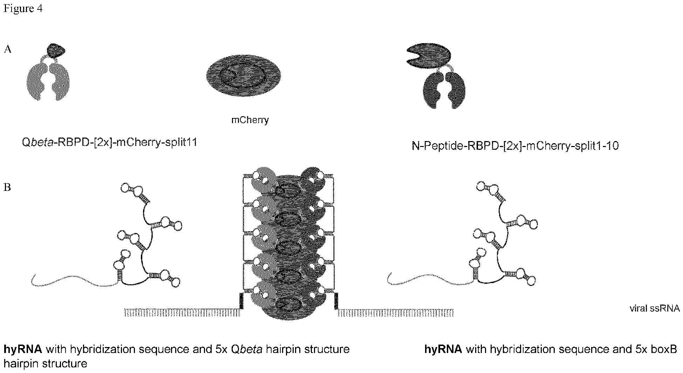

Figure 4 shows another preferred embodiment of the hybridization-based detection of nucleic acids according to the invention. A) Split mCherry components; B) Detection of second locus on the same nucleic acid or a different nucleic acid in the same sample is possible by a system based on split mCherry (schematic depiction). The system requires two probes to specifically hybridize.

### Examples

### Use of the HyEdit system with Ms2-FokI as protein component

### 1. Protocol for in vitro cleavage of a target DNA, such as a PCR product or a plasmid

The Ms2-FokI protein with a TEV-protease-cleavable Strep-tag is overexpressed in *E. coli,* and purified with a tag-specific Strep-Tactin column. The protein is eluted by TEV protease cleavage. Purified Ms2-FokI protein is stored at -80°C in glycerol-containing storage buffer. For cleavage of the target DNA, the target DNA is first resuspended at 100 nM in 30µl reaction buffer (e.g. 10 mM Tris-HCl pH 7.5, 50 mM NaCl, 1 mM DTT, 0.1 mg/ml BSA, 1 mM MgCl2). Two hyRNAs are added in 50nM concentration each. The two hyRNA molecules are designed to hybridize with sites of the target DNA in a head-to-head orientation in a way that the containing Ms2-hairpins are oriented towards each other with ten nucleotides distance between them. The hyRNA molecules are annealed to target DNA by heating the sample to 95°C for 5s and a stepwise temperature decrease to 25°C at 0.1°C per second. The Ms2-FokI protein component is then added at 100nM. The sample is incubated at 37°C for defined intervals from 5 s to 24 h. Samples are heated to 80°C for 5 min for protein denaturation (heat-inactivation and DNA release). Samples are then analysed on 1% agarose gels with Ethidium Bromide by gel electrophoresis to separate DNA of different sizes and quantify cleavage efficiency.

### 2. Protocol for in vivo genome editing:

Approximately 0.5µg of the purified Ms2-FokI protein component is mixed with hyRNA molecules in an equimolar ratio in PBS buffer. An RNA-protein complex will form. The RNA-protein complex mixture is transfected into cells together with 1µg of a donor DNA template (a PCR product in 2-fold excess) by electroporation (e.g. using a Nucleofector machine according to manufacturer instructions, and using 1- to 10-times 10^6 cells). Cells are recovered for at least 24h growth, and editing success can be evaluated e.g. by DNA sequencing or PCR.

Alternatively, a plasmid with genes for the expression of the Ms2-FokI protein and hyRNAs can be delivered by electroporation together with a donor DNA. The donor DNA can also be part of the plasmid DNA itself.

Using a plasmid and/or a donor DNA that additionally encodes a selection marker, such as an antibiotic resistance, allow for the positive selection of cells which were successfully transfected with plasmid and/or donor DNA, and thus have a high chance for being edited.

Alternative to electroporation, other methods for delivery of protein-RNA complexes and/or DNA into cells include injection into cells with a syringe, viral transfection (e.g. adeno- or lentivirus for mammalian cells and Agrobacterium tumefaciens for plant cells), lipofection, receptor internalization and bombardment of cells with metal beads coated with transfection reagents.

With respect to conditions for cell culture and equipment and machines as used, see for example the Methods sections "Plasmid cleavage assay" and "Cellular plasmid cleavage assay" in Schierling B, et al. (A novel zinc-finger nuclease platform with a sequence-specific cleavage module. Nucleic Acids Res. 2012;40(6):2623-2638. doi:10.1093/nar/gkr1112), as incorporated herewith by reference.

### 3. Use of the method for SARS CoV2 ssRNA detection

For viral ssRNA detection, a mouth swab sample from a subject to be diagnosed is diluted in pure water to yield a minimum of 10µl sample. The sample is divided into 5µl aliquots for test and control. For the test, the HyDetect components and RNase inhibitor are added, and the viral ssRNA is detected with a dual fluorophore system (see, for example, figures 3 and 4). For the control, RNase is added, heat inactivated, and the HyDetect components and RNase inhibitor are added in order to detect background noise.

### References as cited

Adli, M. (2018). The CRISPR tool kit for genome editing and beyond. Nature communications, 9(1), 1-13.
Bertrand, E., Chartrand, P., Schaefer, M., et al. (1998). Localization of ASH1 mRNA particles in living yeast. Molecular cell, 2(4), 437-445.
Bitinaite, J., Wah, D. A., Aggarwal, A. K., and Schildkraut, I. (1998). FokI dimerization is required for DNA cleavage. Proceedings of the national academy of sciences, 95(18), 10570-10575.
Boch, J., Scholze, H., Schornack, S., et al. (2009). Breaking the code of DNA binding specificity of TAL-type III effectors. Science, 326(5959), 1509-1512.
Cai, Z., Gorin, A., Frederick, R., Ye, X., Hu, W., Majumdar, A.and Patel, D. J. (1998). Solution structure of P22 transcriptional antitermination N peptide-box B RNA complex. Nature structural biology, 5(3), 203-212.
Cheong, C. G., and Hall, T. M. T. (2006). Engineering RNA sequence specificity of Pumilio repeats. Proceedings of the National Academy of Sciences, 103(37), 13635-13639. Golmohammadi, R., Valegard, K., Fridborg, K., and Liljas, L. (1993). The refined structure of bacteriophage MS2 at 2.8 A resolution. Journal of molecular biology, 234(3), 620-639.
Gott, J. M., Wilhelm, L. J., and Olke, C. U. (1991). RNA binding properties of the coat protein from bacteriophage GA. Nucleic acids research, 19(23), 6499-6503.
Guenther, D. C., Anderson, G. H., Karmakar, S., et al. (2015). Invader probes: Harnessing the energy of intercalation to facilitate recognition of chromosomal DNA for diagnostic applications. Chemical science, 6(8), 5006-5015.
Hegge, J. W., Swarts, D. C., Chandradoss, S. D., et al. (2019). DNA-guided DNA cleavage at moderate temperatures by Clostridium butyricum Argonaute. Nucleic acids research, 47(11), 5809-5821.
Horiya, S., Inaba, M., Koh, C. S., et al. (2009). Replacement of the λ boxB RNA-N peptide with heterologous RNA-peptide interactions relaxes the strict spatial requirements for the formation of a transcription anti-termination complex. Molecular microbiology, 74(1), 85-97.
Kim, Y. G., Cha, J., and Chandrasegaran, S. (1996). Hybrid restriction enzymes: zinc finger fusions to Fok I cleavage domain. Proceedings of the National Academy of Sciences, 93(3), 1156-1160.
LeCuyer, K. A., Behlen, L. S., and Uhlenbeck, O. C. (1995). Mutants of the bacteriophage MS2 coat protein that alter its cooperative binding to RNA. Biochemistry, 34(33), 10600-10606. Lim, F., Spingola, M., and Peabody, D. S. (1994). Altering the RNA binding specificity of a translational repressor. Journal ofBiological Chemistry, 269(12), 9006-9010.
Lim, F., Spingola, M., and Peabody, D. S. (1996). The RNA-binding site of bacteriophage Qβ coat protein. Journal of Biological Chemistry, 271(50), 31839-31845.
Lim, F., Downey, T. P., and Peabody, D. S. (2001). Translational repression and specific RNA binding by the coat protein of the Pseudomonas phage PP7. Journal of Biological Chemistry, 276(25), 22507-22513.
Lim, F., and Peabody, D. S. (2002). RNA recognition site of PP7 coat protein. Nucleic acids research, 30(19), 4138-4144.
Makarova, K. S., Wolf, Y. I., and Koonin, E. V. (2018). Classification and nomenclature of CRISPR-Cas systems: where from here?. The CRISPR journal, 1(5), 325-336.
Miller, J. C., Patil, D. P., Xia, D. F., et al. (2019). Enhancing gene editing specificity by attenuating DNA cleavage kinetics. Nature biotechnology, 37(8), 945-952.
Moscou, M. J., and Bogdanove, A. J. (2009). A simple cipher governs DNA recognition by TAL effectors. Science, 326(5959), 1501-1501.
Obika, S., Nanbu, D., Hari, Y., et al. (1997). Synthesis of 2'-O, 4'-C-methyleneuridine andcytidine. Novel bicyclic nucleosides having a fixed C3,-endo sugar puckering. Tetrahedron Letters, 38(50), 8735-8738.
Peabody, D. S. (1993). The RNA binding site of bacteriophage MS2 coat protein. The EMBO journal, 12(2), 595-600.
Ramirez, C. L., Certo, M. T., Mussolino, C., et al. (2012). Engineered zinc finger nickases induce homology-directed repair with reduced mutagenic effects. Nucleic acids research, 40(12), 5560-5568.
Saito, H., Kobayashi, T., Hara, T., Fujita, Y., Hayashi, K., Furushima, R., and Inoue, T. (2010). Synthetic translational regulation by an L7Ae-kink-turn RNP switch. Nature chemical biology, 6(1), 71.
Sau, S. P., Kumar, T. S., and Hrdlicka, P. J. (2010). Invader LNA: efficient targeting of short double stranded DNA. Organic and biomolecular chemistry, 8(9), 2028-2036.
Sau, S. P., Madsen, A. S., Podbevsek, P., et al. (2013). Identification and characterization of second-generation invader locked nucleic acids (LNAs) for mixed-sequence recognition of double-stranded DNA. The Journal of organic chemistry, 78(19), 9560-9570.
Simeonov, A., and Nikiforov, T. T. (2002). Single nucleotide polymorphism genotyping using short, fluorescently labeled locked nucleic acid (LNA) probes and fluorescence polarization detection. Nucleic acids research, 30(17), e91-e91.
Singh, S. K., Koshkin, A. A., Wengel, J., and Nielsen, P. (1998). LNA (locked nucleic acids): synthesis and high-affinity nucleic acid recognition. Chemical communications, (4), 455-456. Van Gilst, M. R., Rees, W. A., Das, A., et al. (1997). Complexes of N antitermination protein of phage λ with specific and nonspecific RNA target sites on the nascent transcript. Biochemistry, 36(6), 1514-1524.
Wang, J., Friedman, G., Doyon, Y., et al. (2012). Targeted gene addition to a predetermined site in the human genome using a ZFN-based nicking enzyme. Genome research, 22(7), 1316-1326. Wu, J., Tang, B., and Tang, Y. (2020). Allele-specific genome targeting in the development of precision medicine. Theranostics, 10(7), 3118.
Xu, S., Cao, S., Zou, B., Yue, Y., et al. (2016). An alternative novel tool for DNA editing without target sequence limitation: the structure-guided nuclease. Genome biology, 17(1), 186.
Zamore, P. D., Williamson, J. R., and Lehmann, R. (1997). The Pumilio protein binds RNA through a conserved domain that defines a new class of RNA-binding proteins. Rna, 3(12), 1421.

## Claims

1. An RNA-guided nuclease or recruitment system comprising:
i) at least one polypeptide selected from the group of a polypeptide comprising a sequence-unspecific endonuclease domain (sNUC), an effector polypeptide, such as, for example a fluorescent protein, and a transcriptional/translational repressor or activator polypeptide,
ii) at least one RNA-binding protein domain (RBPD), wherein the at least one polypeptide, and preferably the sNUC, of i) is complexed with and/or genetically fused to the at least one RBPD to form a polypeptide-RBPD, preferably an sNUC-RBPD, complex or fusion, and
iii) at least one hybridization RNA (hyRNA), comprising at least one RBPD-binding sequence that binds the RBPD of the polypeptide-RBPD, and preferably in the sNUC-RBPD complex or fusion, and a hybridization sequence that binds to a selected target sequence.

2. The RNA-guided nuclease or recruitment system according to claim 1, further comprising a donor DNA molecule comprising a donor DNA sequence flanked by DNA sequences that are homologous to the DNA sequences upstream and downstream to the target sequence.

3. The RNA-guided nuclease system according to any one of claims 1 or 2, wherein the sNUC comprises a non-specific TypeIIS restriction enzyme nuclease domain thereof, for example the FokI endonuclease domain, or PvuII.

4. The RNA-guided nuclease system according to any one of claims 1 to 3, wherein the enzymatically active sNUC is capable of cleaving a double strand in the target sequence, or wherein only one sNUC of an sNUC dimer or multimer is enzymatically active, and provides a nick in the target sequence, wherein preferably cleavage or nicking of the target sequence is independent from the recognition of a protospacer adjacent motif (PAM) in the target sequence.

5. The RNA-guided nuclease or recruitment system according to any one of claims 1 to 4, wherein the RBPD is selected from the group consisting of MS2 bacteriophage coat protein (MCP), PP7 bacteriophage coat protein (PCP), GA bacteriophage coat protein (GCP), Qβ bacteriophage coat protein (QCP), bacteriophage lambda N peptide, Pumilio protein, and an FBF protein or natural/artificial riboswitches.

6. The RNA-guided nuclease or recruitment system according to any one of claims 1 to 5, wherein the RBPD-binding sequence of the hybridization RNA is a hairpin sequence, wherein preferably the hairpin sequence is selected from the group consisting of MS2 RNA hairpin sequence, PP7 RNA hairpin sequence, GA RNA hairpin sequence, Qβ RNA hairpin sequence, boxB hairpin sequence, and PUF binding sequence.

7. A set of nucleic acid molecules, each encoding for at least one polypeptide of the RNA-guided nuclease or recruitment system according to any one of claims 1 to 6, and optionally furthermore encoding for at least one donor DNA molecule according to any one of claims 2 to 6, or a nucleic acid vector or vector system comprising the nucleic acid molecules,

8. The set of nucleic acid molecules or the vector or vector system according to claim 7, further comprising at least one hyRNA molecule according to any one of claims 1 to 6, or a recombinant cell comprising the set of nucleic acid molecules or the vector or vector system according to claim 7, and optionally at least one hyRNA molecule according to any one of claims 1 to 6, wherein preferably the cell is a eukaryotic cell, preferably a mammalian cell.

9. A method for nicking or cutting a selected target nucleic acid sequence in a cell, comprising the steps of:
i) Providing a cell comprising the target sequence to be cleaved;
ii) Introducing the RNA guided nuclease system according to claim 1 into the cell; and binding the hybridization sequence of the hybridization RNA to the selected target sequence; and
iii) Introducing of either a single-strand nick or a double-strand cut in the selected target sequence based on the activated at least one sNUC of the RNA guided nuclease system.

10. A method for in vivo or in vitro genome editing a selected target nucleic acid sequence in a cell, comprising the steps of:
i) Providing a cell comprising the target sequence to be edited;
ii) Introducing the RNA guided nuclease system according to claim 1 or 2 into the cell; and binding the hybridization sequence of the hybridization RNA to the selected target sequence;
iii) Introducing of either a single-strand break or a double-strand break into the selected target sequence based on the activated at least one sNUC of the RNA guided nuclease system; and either
iv) Editing the single-strand break or double-strand break in the selected target sequence without the presence of a DNA donor template comprising a non-homologous end joining (NHEJ), or
v) Editing the single-strand break or double-strand break in the selected target sequence with the presence of a DNA donor template comprising homologous recombination (HR).

11. A method for in vitro or in vitro labelling of a selected target sequence comprising the steps of:
i) Providing a cell comprising the target sequence to be labelled;
ii) Introducing the RNA-guided recruitment system according to claim 1 into the cell;
iii) Binding the hybridization sequence of the hybridization RNA to the selected target sequence; and
iii) Detecting said binding to the selected target sequence based on the effector as recruited to said selected target sequence.

12. A method for in vitro or in vitro detecting of a selected target nucleic acid sequence of interest in a cell or sample, comprising the following steps of:
i) Providing a cell or sample comprising the nucleic acid to be detected;
ii) Adding or introducing a first RNA guided recruitment system according to claim 1, wherein said effector protein comprises a non-active bait protein sequence, and wherein the hybridization sequence of the hybridization RNA binds to the selected target sequence of the nucleic acid to be detected;
iii) Adding or introducing a second RNA guided recruitment system according to claim 1, wherein said effector protein comprises a non-activate prey protein sequence, and wherein the second hybridization RNA binds to the selected target sequence of the nucleic acid to be detected and at least one sequence upstream and/or downstream of the selected target sequence, preferably directly or in close proximity to the selected target sequence; and
iv) Detecting of the selected target sequence of interest in the cell or sample if a signal based on an interaction of the bait and the prey protein sequence is detected, wherein preferably the signal as detected is selected from a fluorescence signal and/or the activity of an enzyme.

13. The method according to any one of claims 9 to 12, wherein the selected target sequence is present in the chromosome of the cell, or on an extrachromosomal nucleic acid, such as, for example, a plasmid or other vector, and wherein said sequence is preferably a recombinant sequence.

14. The method according to any one of claims 10 to 13, wherein the RBPD-binding sequence of the hybridization RNA comprises between 1 and 2 hairpins for use in editing, between 1 and 5 hairpin forming sequences for use with regulatory effectors, and between 1 and 20 hairpin forming sequences for use with labelling effectors.

15. Use of the RNA-guided nuclease system according to any one of claims 1 to 6 for nicking or cutting a selected target nucleic acid sequence in a cell or an in vitro sample, or use of the RNA-guided recruitment system according to any one of claims 1 to 6 in vitro or in vitro labelling of a selected target sequence or for in vitro or in vivo detecting of a selected target nucleic acid sequence of interest in a cell or sample, or use of the RNA-guided nuclease system according to any one of claims 2 to 6 for in vivo or in vitro genome editing of a selected target nucleic acid sequence in a cell.
